# EUROPEAN PATENT APPLICATION

(11) **EP 4 285 940 A1**
(43) Date of publication of application: **06.12.2023**
(21) Application number: 23175120.7
(22) Date of filing: 24.05.2023
(51) Int. Cl.: A61L 2/08, A61L 2/10, B08B 1/02, B08B 7/00

(54) **METHOD OF CLEANING CONTAINER PROCESSING SYSTEM AND CONTAINER PROCESSING SYSTEM**

(30) Priority: 03.06.2022 JP 2022091010
(71) Applicant: Pacraft Co., Ltd., Tokyo 108-0014 (JP)
(72) Inventor: Nagata, Keiko, Iwakuni-shi (JP); Yamane, Noriyuki, Iwakuni-shi (JP)
(74) Representative: Grünecker Patent- und Rechtsanwälte PartG mbB

(57) **Abstract**

A method of cleaning a container processing system includes the step of applying a photocatalyst to a facility that the container processing system includes.

## Description

### TECHNICAL FIELD

The present disclosure relates to a method of cleaning a container processing system and a container processing system.

### BACKGROUND ART

Japanese patent application publication No. 2013-193789 discloses a sterilization device that aims to sterilize a packaging material by using a photocatalyst without changing the quality of the packaging material.

### SUMMARY OF THE INVENTION

In a container processing system, cleaning work, such as cleaning and sanitizing devices, for keeping the devices in a clean condition is carried out manually. However, the manual cleaning work of devices requires considerable labor and time.

The present disclosure has been made in view of the above-mentioned circumstances and has an object of providing a technology that is advantageous to reducing the burden of cleaning work for a container processing system.

One aspect of the present disclosure is directed to a method of cleaning a container processing system comprising the step of applying a photocatalyst to a facility that a container processing system includes.

The facility to which the photocatalyst is applied may include a touch panel device.

The container processing system may comprise a control board and a device controlled by the control board, and the facility to which the photocatalyst is applied may include the control board.

The container processing system may comprise: a processing device that performs a processing using a container; and a safety cover that restricts access to the processing device, and the facility to which the photocatalyst is applied may include the safety cover.

The facility to which the photocatalyst is applied may include a processing device that performs a processing using a container.

The facility to which the photocatalyst is applied may include a container conveyance device that conveys a container.

Anther aspect of the present disclosure is directed to a container processing system comprising a facility having a photocatalyst.

The facility having the photocatalyst may include a touch panel device.

The container processing system may comprise a control board and a device controlled by the control board, and the facility having the photocatalyst may include the control board.

The container processing may comprise: a processing device that performs a processing using a container; and a safety cover that restricts access to the processing device, and the facility having the photocatalyst may include the safety cover.

The facility having the photocatalyst may include a processing device that performs a processing using a container.

The facility having the photocatalyst may include a container conveyance device that conveys a container.

According to the present disclosure, it is advantageous to reducing the burden of cleaning work for a container processing system.

### BRIEF DESCRIPTION OF DRAWINGS

Fig. 1 is an oblique perspective diagram of one example of a packaging processing system;
Fig. 2 is an oblique perspective diagram of one example of a packaging processing system;
Fig. 3 is a plan view of one example of a bag;
Fig. 4 is a cross-sectional view showing one example of a touch panel device; and
Fig. 5 is a cross-sectional view showing a schematic configuration of one example of a safety cover.

### DETAILED DESCRIPTION

Below, a packaging processing system that seals a bag after contents are introduced into the bag is described as one example of a container processing system. The technology of the packaging processing system described below may be applied, as appropriate, to other container processing systems (for instance, a packaging processing system that uses containers other than bags or a container processing system that uses containers for any processing other than a packaging processing).

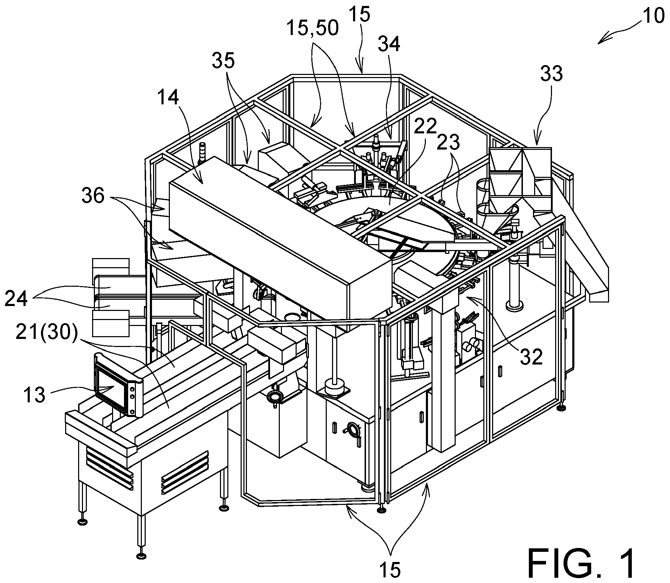

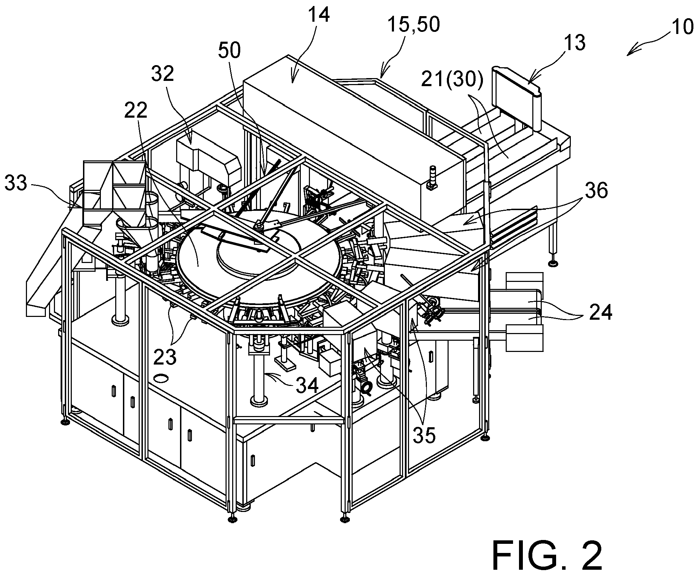

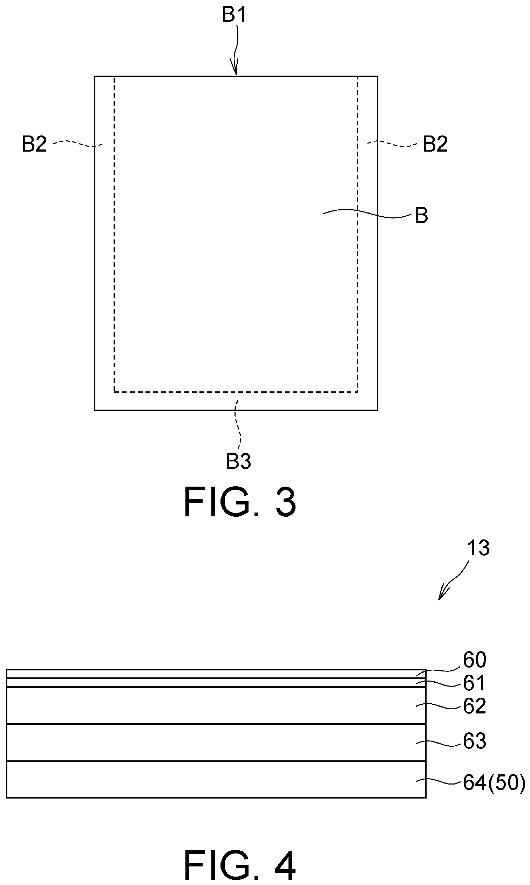

Figs. 1 and 2 are oblique perspective diagrams showing one example of a packaging processing system 10. Fig. 3 is a plan view showing one example of a bag B.

A bag B (i.e., an empty bag) in the present example has a rectangular planar shape as shown in Fig. 3, and has a mouth portion B1 formed by one end part, a bottom portion B3 formed by an end part opposite the mouth portion B1, and both side portions B2 formed by end parts extending between the mouth portion B1 and the bottom portion B3. Each of both side portions B2 and the bottom portion B3 includes a sealed portion and is closed. On the other hand, the mouth portion B1 is formed by an unsealed portion and can be opened and closed freely. The introduction of contents and a gas into a bag B, described below, is done through the mouth portion B1.

The packaging processing system 10 shown in Figs. 1 and 2 comprises carry-in belts 21, a rotary table 22, a plurality of bag support units 23 and carry-out belts 24. Further, the packaging processing system 10 comprises a touch panel device 13 (HMI: Human Machine Interface), a control board 14, a safety cover 15, a mouth opening device 32, a content introduction device 33, a gas injection device 34, heating sealing devices 35, and cooling sealing devices 36.

Each of the carry-in belts 21, the rotary table 22, the bag support units 23 and the carry-out belts 24 works as a container conveyance device to convey bags B.

The carry-in belts 21 work as a storage unit 30 that stores a plurality of bags B (i.e., empty bags) shown in Fig. 3. Bags B on the carry-in belts 21 (in particular, among the plurality of bags B placed on the carry-in belts 21, bags B located at the most downstream and at predetermined take-out positions) are taken out by a take-out device (not shown in the drawings) and are then passed to bag support units 23. The carry-in belts 21 (in particular, the parts on which bags B are placed) runs downstream in accordance with passing the bags B to the bag support units 23, so that respective bags B on the carry-in belts 21 are, in turns, placed at take-out positions, taken out by the take-out device, and then passed to bag support units 23.

In the example shown in Figs. 1 and 2, two carry-in belts 21 are provided and two bags B are passed to two bag support units 23, respectively, at a time. Each bag support unit 23 includes two support opening-closing members (i.e., grippers) that can be opened and closed, and both side portions B2 of a bag B are pinched by these support opening-closing members in such a manner that the bag B is supported in a suspended state by the bag support unit 23. The mouth portion B1 of a bag B supported by a bag support unit 23 is directed upward, and the bottom portion B3 thereof is located below the mouth portion B1.

The rotary table 22 is rotated intermittently by a driving device such as a motor (not shown in the drawings). A plurality of bag support units 23 are attached to the outer periphery of the rotary table 22 at equal intervals (i.e., at equiangular intervals), and move in accordance with the rotation of the rotary table 22 and stop intermittently at a plurality of processing stations, respectively.

In the example shown in Figs. 1 and 2, each bag support unit 23 sequentially and repeatedly goes around a bag reception station, a mouth opening station, a content introduction station, an empty station, a gas injection station, a heating sealing station, and a cooling release station. At each of these stations, two bag support units 23 stop intermittently at a time. Then, at each of the mouth opening station, the content introduction station, the gas injection station, the heating sealing station and the cooling release station, two bags B supported by two bag support units 23 are intermittently stopped at a time, and various processes are performed on the two bags B at a time.

Each bag support unit 23 receives and supports a bag B (i.e., an empty bag) supplied from a carry-in belt 21 (i.e., the storage unit 30) via the take-out device as described above while being intermittently stopping at the bag reception station.

At the mouth opening station, the mouth portions B1 of bags B supported by bag support units 23 are opened by the mouth opening device 32. After that, the bags B move from the mouth opening station to the content introduction station along with the corresponding bag support units 23 in a state where the mouth portions B1 are kept in an open condition.

At the content introduction station, contents are introduced into bags B through their mouth portions B1 in an open condition, by the content introduction device 33. The type and form of contents are not limited. The contents may be, for instance, food or daily necessities such as detergent. Further, the contents may, for instance, be a solid matter, a liquid matter, or contain both a solid matter and a liquid matter.

No processing is performed on bags B at the empty station. A bag B supported by each bag support unit 23 is intermittently conveyed without being processed at the empty station, to pass through the empty station.

At the gas injection station, a gas is injected into bags B through their mouth portions B1 in an open condition, by the gas injection device 34. The gas supplied by the gas injection device 34 is not limited, and an inert gas (such as nitrogen), water vapor, or oxygen may be injected into bags B by the gas injection device 34.

At the heating sealing station, the mouth portions B1 of bags B in a closed condition are heat-sealed by the heating sealing devices 35 while being pressurized. The distance between the support opening-closing members of a bag support unit 23 that support both side portions B2 of a bag B, may be adjusted in accordance with the opening and closing of the mouth portion B1. The distance between the support opening-closing members may be relatively small while the mouth portion B1 is being maintained in an open state, and the distance between the support opening-closing members may be relatively large while the mouth portion B1 is being maintained in a closed state. In this case, prior to the mouth portion B1 undergoing the above heating sealing process, the mouth portion B1 is closed by enlarging the distance between the support opening-closing members in such a manner that the distance is made to be relatively large.

At the cooling release station, the mouth portions B1 (in particular, heat-sealed parts) of bags B in a closed state are cooled by the cooling sealing devices 36 to stabilize the sealing condition of the mouth portions B1. After that, bags B (i.e., product bags) with contents sealed therein are released from bag support units 23 and the cooling sealing devices 36 to fall down, land on the carry-out belts 24, and are then sent to a subsequent stage by the carry-out belts 24.

The above-described container conveyance device and processing devices included in the packaging processing system 10 (i.e., the carry-in belts 21, the rotary table 22, the bag support units 23, the carry-out belts 24, the mouth opening device 32, the content introduction device 33, the gas injection device 34, the heating sealing devices 35, and the cooling sealing devices 36, for instance) may be suitably realized by any known devices, and the explanation of the specific configuration of such devices is omitted here.

The container conveyance device and the processing devices included in the packaging processing system 10 (at least one of the carry-in belts 21, the rotary table 22, the bag support units 23, the carry-out belts 24, the mouth opening device 32, the content introduction device 33, the gas injection device 34, the heating sealing devices 35, and the cooling sealing devices 36, for instance) are driven under control of the control board (control unit) 14.

The touch panel device 13 is connected to the control board 14. The touch panel device 13 presents various information provided by the control board 14 to users or give instructions input by users, to the control board 14. In response to the touch panel operation conducted by users, the touch panel device 13 visually provides the users with various information about the container conveyance device and the processing devices that are controlled by the control board 14 or transmits instructions input by the users to the control board 14. The control board 14 may change the control of the container conveyance device and the processing devices in accordance with user's instructions provided from the touch panel device 13.

The packaging processing system 10 further comprises a safety cover 15 that restricts access to at least part of the above-mentioned processing devices. The safety cover 15 shown in Figs. 1 and 2 is provided to cover the conveyance path (in the present example, a circular path ) of bags B that move with the bag support units 23, from the outside in the horizontal direction. Each bag B moves intermittently along with a bag support unit 23 in the inner space enclosed by the safety cover 15 and undergoes processes by various processing devices (for instance, the mouth opening device 32, the content introduction device 33, the gas injection device 34, the heating sealing devices 35 and the cooling sealing devices 36).

The safety cover 15 in the present example includes beam portions located above the conveyance path for bags B that move along with the bag support units 23, and an excitation light source 50 is installed on the beam portions. The excitation light source 50 emits an excitation light (for instance, a light including ultraviolet light) that can excite a photocatalyst described later, toward a facility of the packaging processing system 10 (e.g., the mouth opening device 32, the content introduction device 33, the gas injection device 34, the heating sealing devices 35 and the cooling sealing devices 36).

The packaging processing system 10 in the present embodiment described above comprises a facility including a photocatalyst, and cleaning effects are exerted by the photocatalyst receiving the excitation light.

The photocatalyst referred to here can generally mean substances that can generally enhance cleaning effects by being irradiated with an excitation light. Such a photocatalyst is not limited, and as an example, substances (e.g., titanium dioxide and tungsten oxide) that enhance oxidizing effects and/or hydrophilic effects in response to the irradiation of an excitation light, may be used as such a photocatalyst. For instance, by irradiating a titanium dioxide used as a photocatalyst with ultraviolet light, the titanium dioxide can cause oxidative decomposition of the attached stains (e.g., organic substances, etc.) and further can promote the formation of a film of water given to its surface (i.e., the formation of a water film) and wash off stains by the water film.

A facility having a photocatalyst A may be given the photocatalyst in an arbitrary manner.

For instance, by applying a photocatalyst to a facility included in the packaging processing system 10, the facility may have the photocatalyst. By applying or spraying a liquid photocatalyst-containing material that contains a photocatalyst onto a surface of a facility, the application of the photocatalyst onto the facility may be carried out. The surface of the facility having received the application of the photocatalyst in this manner is partially or entirely coated by a photocatalytic layer containing the photocatalyst.

Alternatively, a facility having a photocatalyst may inherently contain the photocatalyst. For instance, a facility may congenitally contain a photocatalyst by having the photocatalyst mixed and contained in advance into the constituent material of the facility. As described above, the material included in a facility may already contain a photocatalyst at the time of manufacture of the facility and prior to assembly of the packaging processing system 10.

A facility of the packaging processing system 10 to which a photocatalyst is imparted (e.g., applied) is not limited. For instance, the touch panel device 13, the processing devices that perform processing using bags B (i.e., containers) (e.g., the mouth opening device 32, the content introduction device 33, the gas injection device 34, the heating sealing devices 35 and the cooling sealing devices 36), the safety cover 15, and the container conveyance devices that convey bags B (e.g., the carry-in belts 21, the rotary table 22, the bag support units 23 and the carry-out belts 24) may be included in the facility having a photocatalyst.

In particular, in cases where a facility that is easily soiled or has a large impact when being soiled have a photocatalyst, the benefits brought about by the cleaning effects of the photocatalyst tend to be large. For instance, a photocatalyst may be given to some or all of a facility with which people potentially come in contact (for instance, a facility that people might come in contact with during adjustment, maintenance or inspection of the facility), in particular to sections of the facility that include parts that people frequently make contact with. Further, a photocatalyst may be given to some or all of a facility to which contents may adhere (for instance, of a facility to which contents flying out of a facility or bags may adhere) before, during, or after the introduction of the contents into bags.

As described above, according to the present embodiment, by virtue of the cleaning effects of a photocatalyst that a facility of the packaging processing system 10 has, the facility can be cleaned without human intervention. Thus, it is possible to reduce the workload of those who perform the cleaning of the container processing system, for instance, reducing the frequency of cleaning operations or lengthening the interval between periodic cleaning operations. Even if it is difficult to keep a facility of the packaging processing system 10 clean properly by the cleaning action of the photocatalyst alone, the cleaning action of the photocatalyst may reduce the workload of manual cleaning, shorten the cleaning work time, or restore the facility to a sufficiently clean state with a simple cleaning operation.

Further, the application of a photocatalyst to a facility of the packaging processing system 10 can reduce the frequency with which people access to the packaging processing system 10 in order to clean the packaging processing system 10. This reduction in the frequency of the need for human presence near the packaging processing system 10 enhances the safety of the cleansing operations for the packaging processing system 10 and further can be advantageous in terms of the automation of the operations of the packaging processing system 10.

Further, the cleaning action of a photocatalyst (in particular, the oxidative decomposition action) can clean the packaging processing system 10 without using water. This can reduce the frequency of cleaning operations using water and thus can effectively suppressing the generation of rust in a facility, and as a result, the shortening of the device life can be curbed.

As just described, according to the present embodiment, it is advantageous in terms of cleaning, sanitizing, antifouling, and rust prevention of the packaging processing system 10 (i.e., the container processing system).

### [Variant examples]

Whereas the excitation light source 50 is installed on the beam portions located in the ceiling portion of the safety cover 15 in the example shown in Figs. 1 and 2, the excitation light source 50 may be installed in any position and form that allows irradiation of a photocatalyst that a facility has with the excitation light.

Therefore, the position of the excitation light source 50 relative to a facility that has a photocatalyst is not limited. The excitation light source 50 may be installed at one or more locations that are upward, downward, and/or horizontally away from a facility having a photocatalyst, and the excitation light from the excitation light source 50 may enter the facility in an upward direction, a downward direction, a horizontal direction and/or an oblique direction (in other words, a direction oblique to the horizontal direction and/or the vertical direction) in a direct manner or an indirect manner. Further, the excitation light source 50 may be provided as part of a facility having a photocatalyst. Further, the excitation light emitted from the excitation light source 50 may be applied to a photocatalyst that a facility has, from outside and/or inside the facility.

Further, light emitted from artificial light sources, such as fluorescent lamps, provided to illuminate the location (e.g., a room) where the packaging processing system 10 is installed, or natural light such as sunlight may be used as an excitation light to excite a photocatalyst that a facility of the packaging processing system 10 has. In such cases, the excitation light source 50 that is provided primarily for the purpose of emitting an excitation light to excite the photocatalyst, may be installed or may not be installed.

Fig. 4 is a cross-sectional diagram showing one example of the touch panel device 13. Fig. 4 merely shows a schematic configuration of the touch panel device 13, and the actual touch panel device 13 may have a different configuration (e.g., a different size ratio) from the touch panel device 13 shown in Fig. 4. In the touch panel device 13 shown in Fig. 4, a liquid crystal panel 63 is provided between a touch panel 62 and a backlight 64, and the touch panel 62 is covered by a protective sheet 61. A photocatalytic coating layer 60 containing a photocatalyst is provided to cover the protective sheet 61. Each of the protective sheet 61, the touch panel 62, the liquid crystal panel 63 and the backlight 64 may have any configuration, and for instance, known devices may be used for them.

Illumination light emitted from the backlight 64 can penetrate the liquid crystal panel 63, the touch panel 62, the protective sheet 61 and the photocatalytic coating layer 60. Specifically, the photocatalytic coating layer 60 can be irradiated with the illumination light from the backlight 64. Therefore, in a case where the illumination light from the backlight 64 includes an excitation light that can excite the photocatalyst contained in the photocatalytic coating layer 60, the backlight 64 can be used as the excitation light source 50.

When operating the touch panel device 13 shown in Fig. 4, a user contacts the photocatalytic coating layer 60. Thus, the user may contaminate the photocatalytic coating layer 60 by contact. On the other hand, by using the backlight 64 as the excitation light source 50, the photocatalyst in the photocatalytic coating layer 60 is excited by the illumination light from the backlight 64 to exert cleaning effects while the user is performing the touch panel operation, which can effectively reduce contamination on the photocatalytic coating layer 60.

In the touch panel device 13 shown in Fig. 4, the photocatalytic coating layer 60 is provided to cover the touch panel 62 via the protective sheet 61, but the touch panel device 13 need not include the protective sheet 61. In this case, the photocatalytic coating layer 60 may be provided to directly cover the touch panel 62.

Further, the touch panel device 13 shown in Fig. 4 has an out-cell type structure in which the touch panel 62 is provided outside the liquid crystal panel 63, but the touch panel 62 may be provided inside the liquid crystal panel 63 and the touch panel 62 may form part of the liquid crystal panel 63. In this case, one or more touch panel function parts that work as the touch panel 62 may be provided outside the liquid crystal layer of the liquid crystal panel 63 (an on-cell type structure) or may be provided within the liquid crystal layer (an in-cell type structure).

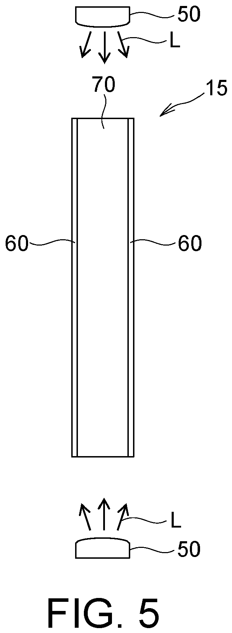

Fig. 5 is a cross-sectional view showing a schematic configuration of one example of the safety cover 15. The safety cover 15 shown in Fig. 5 comprises: a light transmitting partition 70 that can transmit the excitation light L emitted from excitation light sources 50; and photocatalytic coating layers 60 that cover the front surface and the back surface of the light transmitting partition 70. The excitation light L emitted from the excitation light sources 50 that are respectively provided above and below the light transmitting partitions 70, enters the interior of the light transmitting partition 70. The excitation light L that has entered the interior of the light transmitting partition 70 travels inside the light transmitting partition 70, and is reflected at the boundary surfaces between the light transmitting partition 70 and the photocatalytic coating layers 60 and is emitted from the boundary surfaces.

The excitation light L emitted from the inside of the light transmitting partition 70 and entering a photocatalytic coating layer 60 is applied to the photocatalyst contained in the photocatalytic coating layer 60 to excite the photocatalyst. As a result, the photocatalytic coating layer 60 exhibits a cleaning effect. By irradiating the photocatalytic coating layers 60 from the inside of the safety cover 15 (a facility) with the excitation light in this way, the excitation light can be efficiently applied to the photocatalytic coating layers 60 without the excitation light being blocked by dirt adhering to the outer surfaces (the exposed surfaces) of the photocatalytic coating layers 60.

The configuration of the safety cover 15 shown in Fig. 5 can be applied to any other facility. Specifically, in cases where a facility having a photocatalyst comprises a light transmissive body that can transmit the excitation light L and a photocatalytic coating layer 60 (a photocatalyst) that is provided to cover part or the entire of the light transmissive body, the photocatalytic coating layer 60 can be irradiated with the excitation light L from the inside of the facility.

The present disclosure is not limited to the embodiments and variations described above. For instance, various modifications may be added to each element of the above-described embodiments and variations, and configurations may be partially or entirely combined among the above-described embodiments and variations. Further, the effects produced by the present disclosure are not limited to the effects described above, and effects specific to the specific configuration of each embodiment may also be produced. As described above, various additions, modifications and partial deletions may be made to each element described in the claims, specification, and drawings to the extent that they do not depart from the technical concept and the spirit of the present disclosure.

### [Additional notes]

The present disclosure may also adopt the following configurations.

### [Item 1]

A method of cleaning a container processing system comprising the step of applying a photocatalyst to a facility that a container processing system includes.

### [Item 2]

The method of cleaning a container processing system as defined in item 1, wherein the facility to which the photocatalyst is applied includes a touch panel device.

### [Item 3]

The method of cleaning a container processing system as defined in item 1 or 2,
wherein the container processing system comprises a control board and a device controlled by the control board, and
wherein the facility to which the photocatalyst is applied includes the control board.

### [Item 4]

The method of cleaning a container processing system as defined in any one of items 1 to 3,
wherein the container processing system comprises: a processing device that performs a processing using a container; and a safety cover that restricts access to the processing device, and
wherein the facility to which the photocatalyst is applied includes the safety cover.

### [Item 5]

The method of cleaning a container processing system as defined in any one of items 1 to 4, wherein the facility to which the photocatalyst is applied includes a processing device that performs a processing using a container.

### [Item 6]

The method of cleaning a container processing system as defined in any one of items 1 to 5, wherein the facility to which the photocatalyst is applied includes a container conveyance device that conveys a container.

### [Item 7]

A container processing system comprising a facility having a photocatalyst.

### [Item 8]

The container processing system as defined in item 7, wherein the facility having the photocatalyst includes a touch panel device.

### [Item 9]

The container processing system as defined in item 7 or 8, comprising a control board and a device controlled by the control board,
wherein the facility having the photocatalyst includes the control board.

### [Item 10]

The container processing system as defined in any one of items 7 to 9, comprising: a processing device that performs a processing using a container; and a safety cover that restricts access to the processing device,
wherein the facility having the photocatalyst includes the safety cover.

### [Item 11]

The container processing system as defined in any one of items 7 to 10, wherein the facility having the photocatalyst includes a processing device that performs a processing using a container.

### [Item 12]

The container processing system as defined in any one of items 7 to 11, wherein the facility having the photocatalyst includes a container conveyance device that conveys a container.

## Claims

1. A method of cleaning a container processing system comprising the step of applying a photocatalyst to a facility that the container processing system includes.

2. The method of cleaning a container processing system as defined in claim 1, wherein the facility to which the photocatalyst is applied includes a touch panel device.

3. The method of cleaning a container processing system as defined in claim 1 or 2,
the container processing system comprises a control board and a device controlled by the control board, and
wherein the facility to which the photocatalyst is applied includes the control board.

4. The method of cleaning a container processing system as defined in any one of claims 1 to 3,
wherein the container processing system comprises: a processing device that performs a processing using a container; and a safety cover that restricts access to the processing device, and
wherein the facility to which the photocatalyst is applied includes the safety cover.

5. The method of cleaning a container processing system as defined in any one of claims 1 to 4, wherein the facility to which the photocatalyst is applied includes a processing device that performs a processing using a container.

6. The method of cleaning a container processing system as defined in any one of claims 1 to 5, wherein the facility to which the photocatalyst is applied includes a container conveyance device that conveys a container.

7. A container processing system comprising a facility having a photocatalyst.

8. The container processing system as defined in claim 7, wherein the facility having the photocatalyst includes a touch panel device.

9. The container processing system as defined in claim 7 or 8, comprising a control board and a device controlled by the control board,
wherein the facility having the photocatalyst includes the control board.

10. The container processing system as defined in any one of claims 7 to 9, comprising: a processing device that performs a processing using a container; and a safety cover that restricts access to the processing device,
wherein the facility having the photocatalyst includes the safety cover.

11. The container processing system as defined in any one of claims 7 to 10, wherein the facility having the photocatalyst includes a processing device that performs a processing using a container.

12. The container processing system as defined in any one of claims 7 to 11, wherein the facility having the photocatalyst includes a container conveyance device that conveys a container.
